# EUROPEAN PATENT SPECIFICATION

(11) **EP 3 013 354 B1**
(45) Date of publication and mention of the grant of the patent: **19.08.2020**
(21) Application number: 14740936.1
(22) Date of filing: 15.01.2014
(51) Int. Cl.: A61K 31/473, A61K 41/00, A61K 47/55, A61P 9/00, A61P 29/00

(54) **COMPOSITION FOR DECREASING INFLAMMATORY RESPONSE IN A BLOOD VESSEL**
ZUSAMMENSETZUNG ZUR VERRINGERUNG DER ENTZÜNDUNGSREAKTION IN EINEM BLUTGEFÄSS
COMPOSTION POUR DIMINUER DE LA RÉPONSE INFLAMMATOIRE DANS UN VAISSEAU SANGUIN

(30) Priority: 16.01.2013 US 201361753203 P
(43) Date of publication of application: 04.05.2016
(73) Proprietor: Alumend, LLC, Sioux Falls, SD 57107 (US)
(72) Inventor: DOWNEY, Therese, J., Brookings, SD 57033 (US); UTECHT, Ronald, E., Madison, SD 57042 (US); MUNGER, Karen, A., Sioux Falls, SD 57105 (US)
(74) Representative: V.O.
(86) International application number: PCT/US2014/011642
(87) International publication number: WO 2014/113450

(56) References cited:
- WO-A2-2007/030939
- WO-A2-2009/126606
- US-A1- 2005 281 883
- US-B1- 6 410 505
- US-B1- 6 609 014
- US-B2- 7 947 839
- US-B2- 8 242 114
- KAIA L. KLOSTER ET AL: "Preferential localization of varying forms of photoactive 1,8-naphthalimide compounds within the atheromatous arterial wall", LASERS IN SURGERY AND MEDICINE, vol. 26, no. 3, 1 January 2000 (2000-01-01), pages 316-322, XP55110655, ISSN: 0196-8092, DOI: 10.1002/(SICI)1096-9101(2000)26:3<316::AID -LSM10>3.0.CO;2-U
- SULLIVAN, GW ET AL.: 'The Role of Inflammation in Vascular Diseases.' JOURNAL OF LEUKOCYTE BIOLOGY. vol. 67, May 2000, pages 591 - 602, XP008181074

## Description

### Field of the Invention

The present invention is directed to a composition comprising a 4-amino-1,8-naphthalimide compound for use in a method of decreasing an inflammatory response in a blood vessel wherein a bolus of a composition comprising a 4-amino-1,8-naphthalimide compound is applied to the damaged blood vessel, wherein the damaged blood vessel is inflamed.

### Background of the Invention

Inflammation is a localized body response to tissue damage. It signifies the body's attempt to defend itself against a pathogen and/or cell/tissue damage and prevents it from spreading and damaging other tissue. Injured cells, basophils, mast cells, and others release histamine and other chemicals that dilate blood vessels, increase blood supply to the injured area, and increase the permeability of local capillaries

The process of acute inflammation is initiated by cells already present in all tissues, mainly resident macrophages, dendritic cells, histocytes, Kupffer cells and mastocytes. These cells present on their surfaces certain receptors named pattern recognition receptors (PRRs), which recognize molecules that are broadly shared by pathogens but distinguishable from host molecules. At the onset of an infection, burn, or other injury, these cells undergo activation and release inflammatory mediators responsible for the clinical signs of inflammation. Vasodilation and its resulting increased blood flow causes redness and increased heat. Increased permeability of the blood vessels results in an exudation (leakage) of plasma proteins and fluid into the tissue, which manifests itself as swelling. The mediator molecules also alter the blood vessels to permit the migration of leukocytes, mainly neutrophils, outside of the blood vessels into the tissue. The neutrophils migrate along a chemotactic gradient created by the local cells to reach the site of injury.

In addition to cell-derived mediators, several acellular biochemical cascade systems consisting of preformed plasma proteins act in parallel to initiate and propagate the inflammatory response. These include the complement system activated by bacteria and the coagulation and fibrinolysis system activated by necrosis, e.g. a burn or a trauma.

The acute inflammatory response requires constant stimulation to be sustained. Inflammatory mediators have short half lives and are quickly degraded in the tissue. Hence, acute inflammation ceases once the stimulus has been removed. The inflammatory response must be actively terminated when no longer needed to prevent unnecessary damage to tissues. Failure to do so results in chronic inflammation, and cellular destruction.

Atherosclerosis actually involves an ongoing inflammatory response. Atherosclerosis affects the entire artery tree, but mostly larger, high-pressure vessels such as the coronary, renal, femoral, cerebral, and carotid arteries. Atherosclerosis is initiated by inflammatory processes in the vessel wall in response to retained low-density lipoprotein (LDL) molecules. Once inside the vessel wall, LDL molecules become susceptible to oxidation by free radicals, and become toxic to the cells. The damage caused by the oxidized LDL molecules triggers a cascade of immune responses, which over time can produce an atheroma. The LDL molecule is globular shaped with a hollow core to carry cholesterol throughout the body.

The body's immune system responds to the damage to the artery wall caused by oxidized LDL by sending specialized white blood cells (macrophages and T-lymphocytes) to absorb the oxidized-LDL. These white blood cells are not able to process the oxidized-LDL, and ultimately grow then rupture, depositing a greater amount of oxidized cholesterol into the artery wall. This triggers more white blood cells, continuing the cycle.

Eventually, the artery becomes inflamed and cholesterol plaque is formed as a hard cover on the wall of the blood vessel. This hard cover is what causes a narrowing of the artery, reduces the blood flow and increases blood pressure.

Treatment for atherosclerosis may include at least one of statins, niacin, aspirin, vitamins, and surgical intervention such as stents or bypass surgery. Surgical intervention includes several risks such as increasing the trauma to the damaged blood vessel, decreasing the luminal diameter of the damaged blood vessel, increasing the incidence of thrombosis and restenosis, increasing the use of pharmacological agents, and losing native compliance of the blood vessel.

It is accordingly an object of the invention to provide a compound, in the form of a composition, to be administered to a patient in need thereof, wherein the compound will decrease an inflammatory response, decrease vasodilation, and/or decrease the blood supply to a damaged blood vessel and/or decrease the permeability of local capillaries. It is believed that by decreasing an inflammatory response to a damaged blood vessel, then one of ordinary skill in the art would be able to moderate all of the downstream effects of an inflammatory response, such as those associated with atherosclerosis and its treatment.

WO 2007/030939 discloses the use of 1,8-naphthalimide compounds for treating inflammatory disorder of the circulatory system, such as vasculitis.

### SUMMARY OF THE INVENTION

In accordance with the invention, there is disclosed a composition comprising a 4-amino-1,8-naphthalimide compound for use in a method of decreasing an inflammatory response in a damaged blood vessel, wherein a bolus of a composition comprising a 4-amino-1,8-naphthalimide compound is applied to the damaged blood vessel, wherein the damaged blood vessel is inflamed.

The accompanying drawings, illustrate one (several) embodiment(s) of the invention and together with the description, serve to explain the principles of the invention.

### BRIEF DESCRIPTION OF THE DRAWINGS

Figure 1 illustrates a cross-section of a rat carotid artery that has not been damaged and has not been treated with any compounds, i.e., is a control.
Figure 2A illustrates a damaged rat carotid artery after 3 hours that was not treated with any compounds..
Figure 2B illustrates a close-up of the damaged rat carotid artery of Figure 2A.
Figure 3A illustrates a damaged rat carotid artery after 2 weeks that was not treated with any compounds.
Figure 3B illustrates a damaged rat carotid artery after 2 weeks that was treated with a 4-amino-1,8-naphthalimide compound, but without any activating agent.
Figures 4A-C illustrate a damaged rat carotid artery after 2 weeks that was treated with a 4-amino-1,8-naphthalimide compound and an activating agent.
Figure 5 illustrates a damaged rat femoral arty after 3 hours that was treated with a 4-amino-1,8-naphthalimide compound and an activating agent.
Figure 6 illustrates the intimal thickness of various damaged blood vessels.
Figure 7 illustrates the intimal thickness of the combined non-inventive damaged blood vessels and the inventive damaged blood vessel.

### DESCRIPTION OF THE EMBODIMENTS

Reference will now be made in detail to the present embodiment (exemplary embodiments) of the invention, an example(s) of which is (are) illustrated in the accompanying drawings. Wherever possible, the same reference numbers will be used throughout the drawings to refer to the same or like parts.

The present invention is directed to a composition comprising a 4-amino-1,8-naphthalimide compound for use in a method of decreasing an inflammatory response in a damaged blood vessel, wherein a bolus of a composition comprising a 4-amino-1,8-naphthalimide compound is applied to the damaged blood vessel, wherein the damaged blood vessel is inflamed. The method can optionally further comprise a step of activating the compound with a sufficient amount of an activating agent. The activating agent is selected from radiated energy, electromagnetic energy, laser, electric current, electrons, thermal neutrons, and chemicals. The inflammatory response is reduced as compared to a damaged blood vessel that has not been subjected to application of the 4-amino-1,8-naphthalimide compound.

The 4-amino-1,8-naphthalimide may be a compound having a structure selected from the group consisting of: 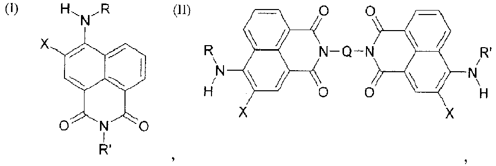 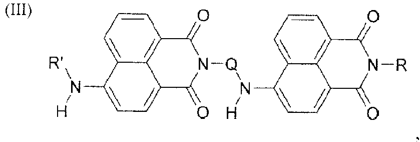 and 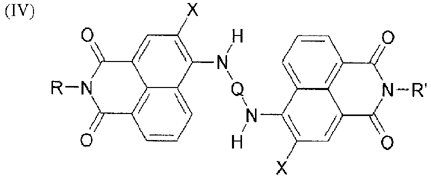 wherein R, R', and Q are each independently selected from the group consisting of straight-chain and branched chain alkyl groups having from 2 to 200 carbons, optionally substituted with one or more ether, amide or amine groups; and wherein X is hydrogen. Naphthalimide compounds which may be used include those described in U.S. Pat. Nos. 5,235,045; 5,565,551; 5,776,600; 5,917,045; 6,410,505; 7,514,399; and 8242,114.

In an aspect, R' can be a substituted alkyl group, wherein the alkyl group is substituted with heteroatoms, such as N, O, P, and S or halogens, such as F, Br, CI, or I. In another aspect, R' can be an amine, a carboxylate, a phosphate, and/or a sulfate.

In an aspect, Q is a polyethylene moiety. Moreover, Q can be a moiety that contains amines and carboxyl groups arranged in a fashion reminiscent of EDTA-like ligands, phosphate groups and/or organic acids arranged in a fashion able to interact with calcium, or functional motifs able to interact with calcium such as luciferin.

In another aspect, Q is an acid or an alcohol, but can also be a thioester, an organophosphorous ester, an anhydride, an amide, a carbamate, or an urea.

In another aspect, the naphthalimide compound has the following structure:

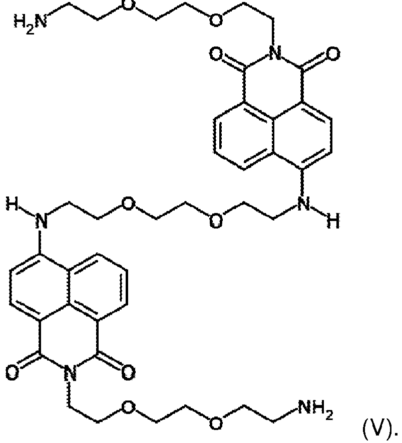

It is hypothesized that upon photoactivation the polyethylene moiety linking the two naphthalimides becomes an intermediate with photoactivated terminal amines. This intermediate has an affinity for binding to amino acid residues on biological molecules, and forms the linkage via a condensation reaction. In particular, the naphthalimide may have a higher affinity for linear protein structures such as collagen or elongated elastin when compared to globular proteins, such as albumin, because the constant twisting and turning of the backbone pulls the hydrogen bonds "out of phase".

The polyether moieties attached in the imide positions impart solubility and the naphthalimide rings are for photoactivation. The solubilizing tails are also believed to mimic a crown ether effect present in known chelating agents. To be clear, however, there is a balancing act to be achieved between solubility and diffusion that must be considered in formulating compounds for use in the present invention.

Below are some additional compounds including a monomer, dimer, and trimer (linear, capped (not shown) and starred (not shown)) of naphthalimide rings. Polymers of the compounds below are also contemplated. Polydispersity of the compounds below are also contemplated.

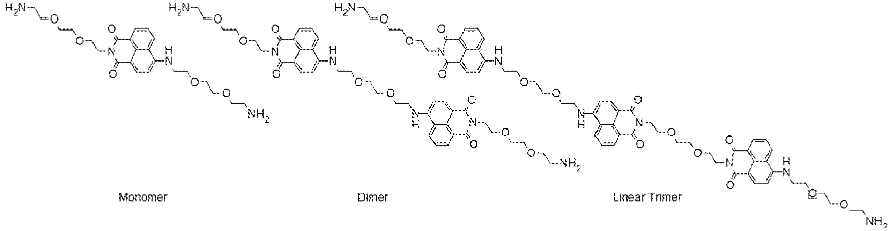

The compounds disclosed herein can be dissolved in a solvent to form a composition. In an example, the solvent can be phosphate buffered saline (PBS). Other suitable solvents include dimethylformamide and isopropyl alcohol. In certain embodiments, the composition can optionally comprise one or more excipients, buffers, carriers, stabilizers, preservatives and/or bulking agents, and is suitable for administration to a patient to achieve a desired effect or result. The composition can be in any desired form, including but not limited to a liquid, a solid, a dispersion, a suspension, a hydrogel, a particle, a nanoparticle, a thin film, and and shaped structure.

The compounds can be present in the composition in a concentration from about 0.01 mg/mL to about 100 mg/mL, for example from about 0.1 mg/mL to about 50 mg/mL, and as a further example from about 2 mg/mL to about 30 mg/mL.

The concentration of the compound can be chosen such that a therapeutic, i.e., a decrease in inflammation, effect is achieved when released into a blood vessel. One of ordinary skill in the art would readily be able to determine the concentration of the compound in order to achieve the desired result.

The composition of the present invention may be provided in vials of various sizes for ease of use. In particular, an 8 mL vial can be used to hold 7 mL of the disclosed composition. The composition can be dispersed from the vial in one dose or is separate doses, for example a first bolus of about 4 mL, followed by a second bolus of about 0.5 to about 1.0 mL. In an aspect, a saline flush can occur between application of the first and second bolus.

It is envisioned that the compound and a composition comprising the compound could be stored in a freeze-dried form, which could be reconstituted with saline/PBS prior to use.

The composition disclosed herein can be applied to a blood vessel. In an aspect, a treatment zone of a blood vessel, such as an artery or vein, can be isolated. In another aspect, the composition is applied in an amount sufficient to provide a high systemic concentration. The composition can be injected into the blood vessel. In an aspect, the blood vessel is the superficial femoral artery (SFA) and its collateral branches. In another aspect, the composition of the present invention is applied to an isolated section of a blood vessel for an extended period of time, such as from about 1 second to about 1 hour, for example from about 1 minute to about 30 minutes, and for example from about 1 minute to about 10 minutes. The amount of time can vary depending upon the damage to the blood vessel and the inflammatory response.

The compositions of the present invention can be used to decrease inflammation, which can improve problems associated with atherosclerosis and treatment thereof.

In an aspect, the composition is delivered by a delivery system comprising an injection port and a treatment zone balloon. A light fiber is in the lumen of the delivery system and is designed to deliver blue light (i.e., 457 nm, for example 450-480 nm, wavelength) at low power. The blue light activates the PEG-based compound to cross-link with biomolecules of the vessel wall, such as collagen.

Any delivery system, including catheter designs with at least one balloon, can be used to deliver the composition to the treatment area, e.g., blood vessel. An exemplary delivery system can be found in U.S. Provisional Application No. 61/679,591, entitled "Endovascular Multi-Balloon Catheters with Optical Diffuser for Treatment of Vascular Stenoses,"

A blood vessel, such as an artery or vein, may be damaged as a result of atherosclerosis, a surgical procedure or repeated access by needle, such as hemodialysis, diabetes, hypertension, autoimmune disease, aneurysm, accident or injury, and a minimally-invasive intervention procedure. Non-limiting examples of surgical procedures include at least one of an endarterectomy, vascular graft implant, vascular anastomosis, and bypass graft. Non-limiting examples of a minimally-invasive procedure comprises at least one of PTA, PTCA, vascular stenting and atherectomy.

An inflammatory response could be established by evidence of at least one of an increase in intimal thickening, clot formation, an increase in concentration of cells, and an increase in mediators. The inflammatory response can be acute or chronic.

The role of cells and mediators in an arterial disease process is well known in the art. For example, white blood cell infiltration (macrophages, platelets, leukocytes, neutrophils, etc.) may be important as well as chemical signaling and thrombosis. Matrix components and inflammatory mediators may be important to the mechanism of action of the 4-amino-1,8-naphthalimide in the healing process of the damaged blood vessel. As discussed above, an increase in the concentration of cells comprises cells chosen from neutrophils, macrophages, mast cells, helper T cells, NKT cells, B cells, fibroblasts, and VSMCs.

The mediators are chosen from matrix metalloproteinase-12, matrix metalloproteinase-3, matrix metalloproteinase-9, chymase, tryptase, IFN-γ, IL-4, MIP-1α, IL-β, TGF-β, immunoglobulins, cathepsins, neutral elastase, matrix metalloproteinase-8, VEGF, MCP-1, and matrix metalloproteinase-2. Matrix metalloproteinases (MMPs) are endopeptidases that degrade matrix proteins, including collagen, and are known to be important in vascular disease. MMPs not only degrade proteins but they also influence cytokine and chemokine activation and may influence cell proliferation, migration, angiogenesis and apoptosis. MMPs are a key player in aneurysm pathology as there are at least 28 different forms. With these and other critical mediators of vascular damage, proteomics can be employed to determine which subtypes are changing with application of 4-amino-1,8-naphthalimide treatment. In addition, real time PCR, ELISA and/or Western blotting techniques may be used to measure these factors.

Without being bound to any particular theory, it is believed that the application of the 4-amino-1,8-naphthalimide compound to an inflamed blood vessel, as described herein, may make the compliance of the vessel more normal, i.e., may restore the vessel to its native compliance. By "native compliance" it is understood to mean the tendency of the blood vessel to expand and contract passively with changes in pressure when not damaged and/or in an inflamed stated.

It is also believed that once the blood vessel exhibits a more native compliance it is likely to exhibit improved healing. One of ordinary skill in the art would be able to assess and determine an improved healing effect by witnessing aspects of a healing response, such as less intimal thickening, the presence of cells and mediators associated with an immune response, etc.

Vessel compliance is an important predictor of vascular disease. A reduction of vessel compliance correlates with the onset of such diseases as atherosclerosis, hypertension, and diabetes mellitus. Thus, the ability to return an inflamed and/or damaged blood vessel to its native compliance may forestall the onset of such diseases and may reduce the need for surgical procedures, such as stents, and/or medications to treat the diseases.

### Examples

### Example 1

The results described below were with the Boston Scientific Maverick balloon catheter, which measured 20 x 1.25-1.5 mm. A 4-amino-1,8-naphthalimide compound was delivered through the catheter and the blood vessel was allowed to soak for 5 minutes before light treatment. The light activation of the compound was with a blue light laser at 450 nm and 100mW for 1 minute. The fiber was 0.02" and was placed in the artery after the balloon damage.

The Medtronic Sprinter Legend balloon catheter, which measures 6 x 1.25 mm was used. The treatment zone was 10 mm. In the Examples, the innermost layer of the artery, the intima, was damaged by inflating the balloon catheter inside the artery and then drawing the inflated balloon down the artery in order to pull the intima away from the medial layer.

The animal protocol has been previously approved by the USD/VAMC IACUC committee and adhered to all published standards and regulations regarding animal experimentation. The VAMC is an AAALAC accredited animal care facility. Male Sprague-Dawley rats weighing between 300-450 grams were anesthetized with either the long acting barbiturate, Inactin, or were anesthetized with isofluroane gas throughout the experiment. For the two-week recovery studies, animals were also given buprenorphene as a pain-management aid for the first few hours of recovery. The carotid and/or the femoral arteries were exposed with a small incision (1-2 cm). Side vessels were clamped or temporarily tied off. The carotid vessel was cannulated through the external carotid artery, which was subsequently tied off after the catheter was removed. The blood flow was maintained through the internal carotid and the contralateral carotids after the procedure. The femoral artery was similarly isolated and side vessels temporarily occluded. The femoral artery was accessed through a popliteal branch, which was subsequently tied off after surgery. Blood flow was maintained through the other popliteal branch and saphenous artery. Three groups were tested: 1) balloon injury only; 2) balloon injury plus the 4-amino-1,8-naphthalimide compound for 5 minutes; and 3) balloon injury plus 4-amino-1,8-naphthalimide compound for 5 minutes followed by light activation for 1 minute. The 4-amino-1,8-naphthalimide compound treatment was administered immediately after damage in all cases.

At 3 hours or 2 weeks after surgery, the rat was killed and damaged carotid and/or femoral arteries and the contralateral control, untouched, arteries were collected from each animal. In most cases, the arteries were drop fixed in 10% buffered formalin for one hour and then 20% glycerol overnight. The arteries were then frozen in OCT compound and sectioned at 7 µm on a cryostat. The slides were then hydrated in PBS for 10 minutes and coverslips were applied with an aqueous mounting media containing DAPI. Sections were viewed with a Zeiss LSM 700 confocal microscope and analyzed with Zen 2011 blue edition software. Photos were taken of representative arteries and are presented below.

These results are from a total of 11 animals. 6 animals were studied at 2 weeks (2 injury only, 1 4-amino-1,8-naphthalimide compound only and 3 with 4-amino-1,8-naphthalimide compound + light activation) and 5 animals were studied after 3 hours (2 injury only, 1 4-amino-1,8-naphthalimide compound only and 2 with 4-amino-1,8-naphthalimide compound + light activation)

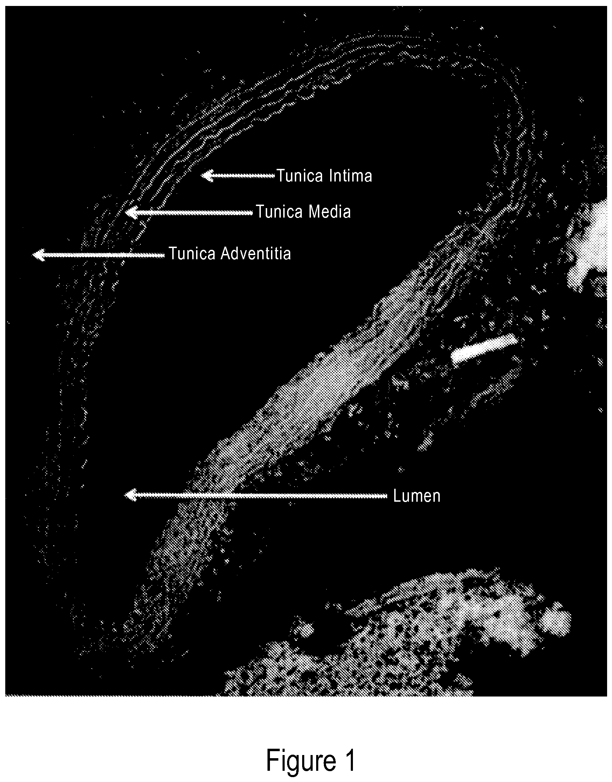

Figure 1 shows the anatomy of the rat artery, in this case, the carotid artery. The media comprises collagen and smooth muscle cells in between layers of elastin. In the Figure, the elastin layers appear as red wavy lines. The outer adventitia is less organized and not as dense. The intimal layer is very thin in normal vessels and can only be seen here as a few cells that are stained blue with the DAPI dye. This Figure represents an untreated control.

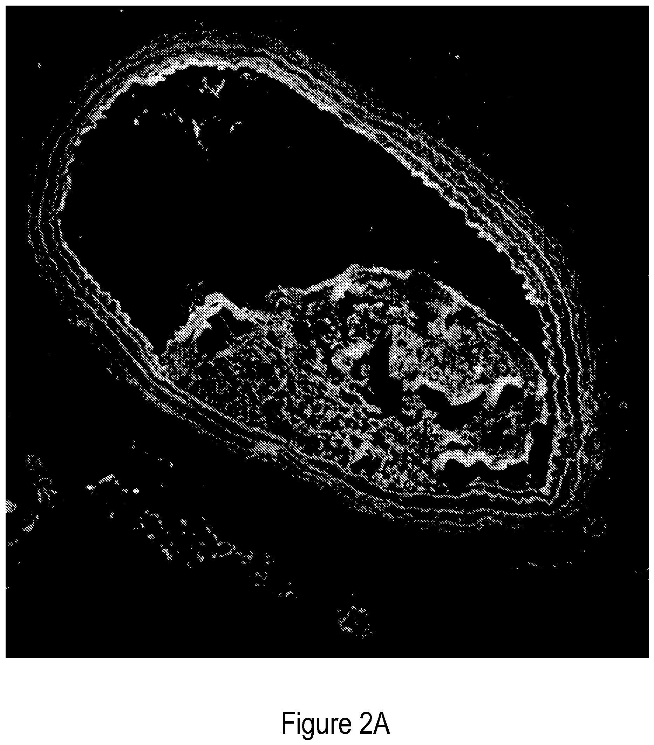

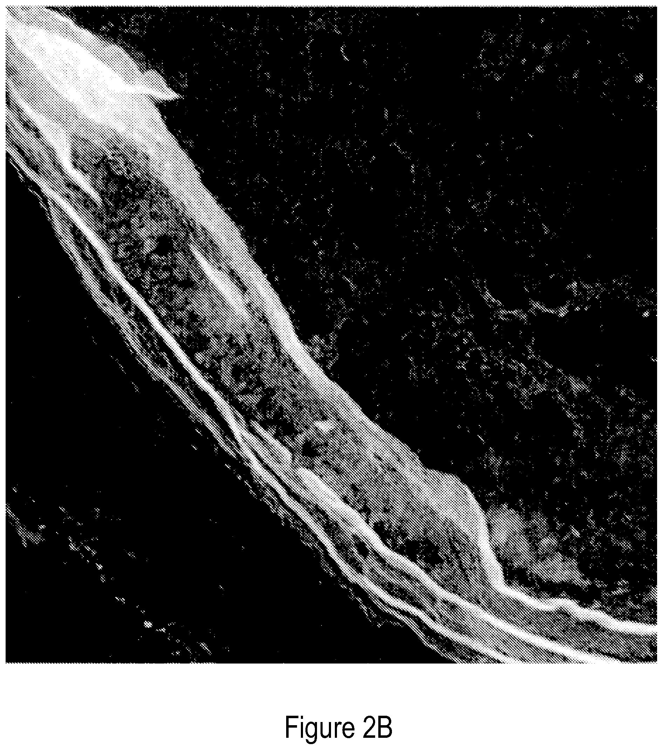

Figure 2A is a damaged artery at 3 hours after the initial damage. The clot formation and the damage to the intima and inner medial layer are visible on the lower right, inner edge of the carotid artery. The clot formation is an indication of the initial inflammatory response. A large amount of infiltrating cells is seen into the medial layer as shown in Figure 2B.

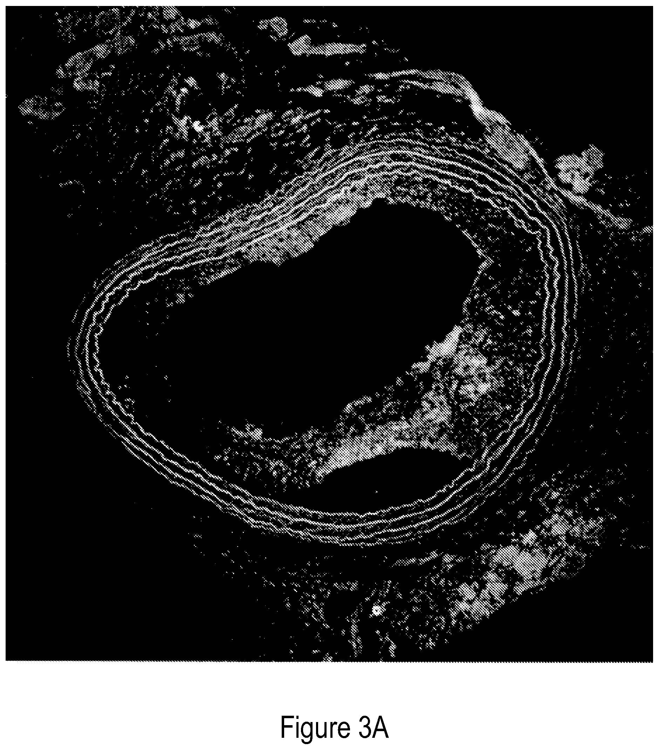

Figure 3A is a damaged artery at 2 weeks after the initial damage. This artery was not treated with the 4-amino-1,8-naphthalimide compound. There is a large amount of intimal thickening due to the inflammatory response.

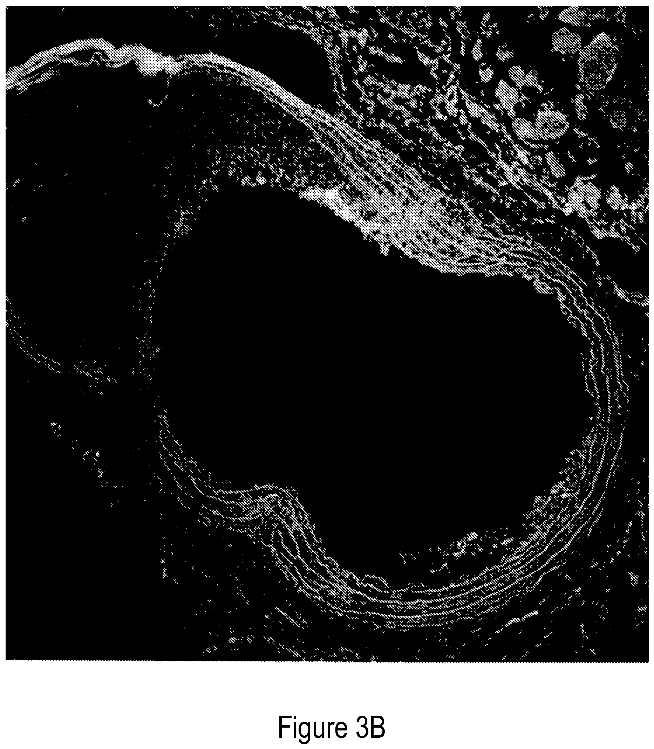

Figure 3B is a damaged artery at 2 weeks after the initial damage. This artery was treated with 4-amino-1,8-naphthalimide compound, but was not subjected to an activating agent. This artery also exhibits a large amount of intimal thickening due to the inflammatory response.

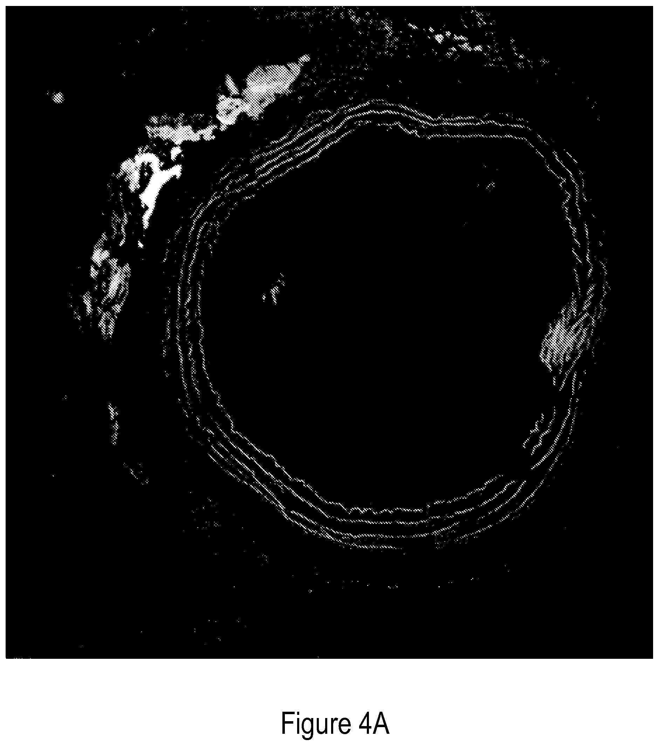

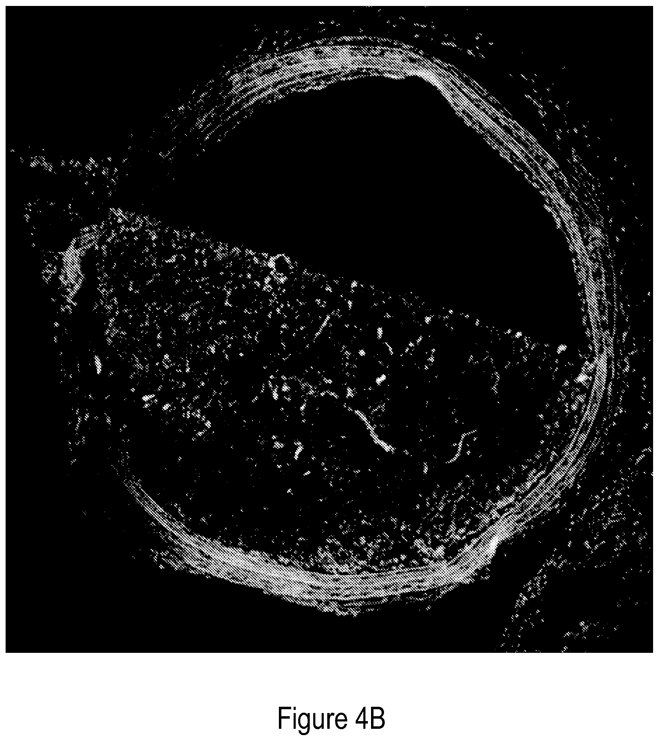

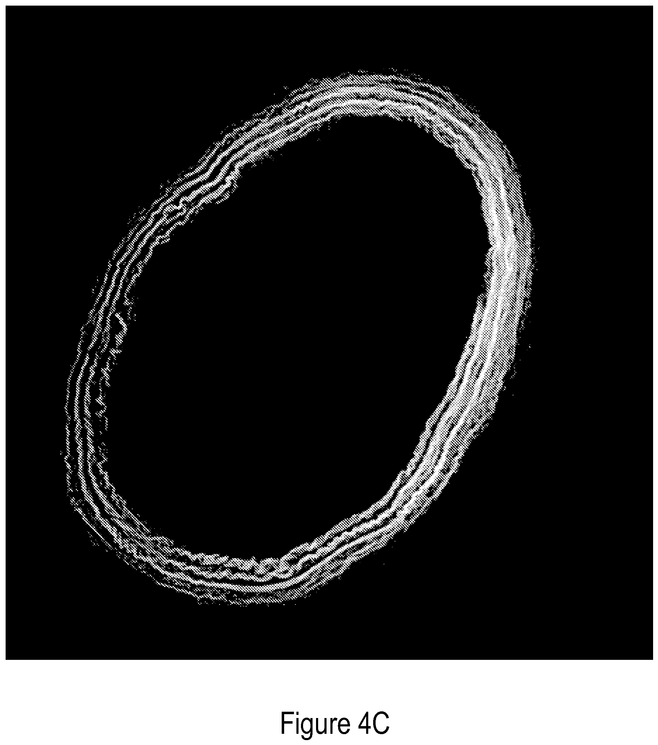

Figures 4A-4C are damaged arteries at 2 weeks after the initial damage. The arteries were treated with 4-amino-1,8-naphthalimide compound and were subjected to an activating agent. The arteries do not exhibit intimal thickening, with the slight exception of Fig. 4C at the arrow.

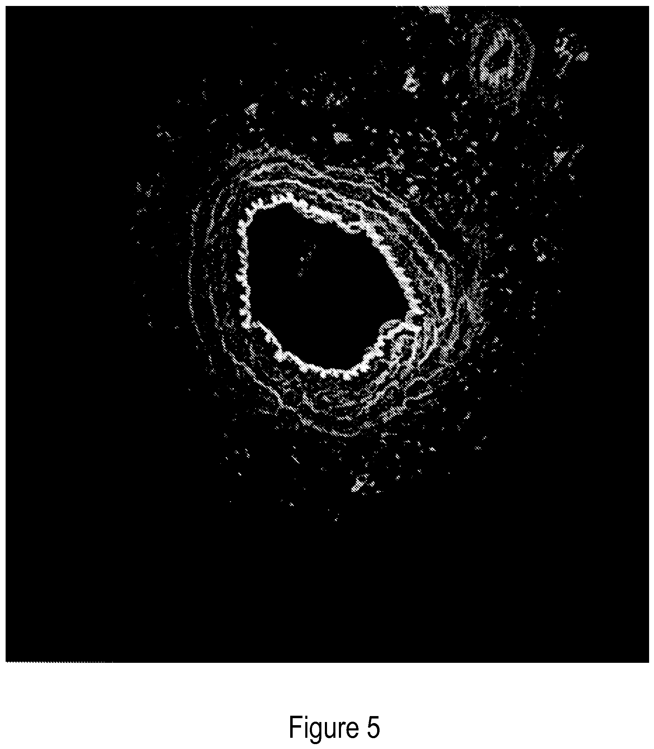

Figure 5 is a damaged femoral artery at 3 hours after the initial damage. This artery was treated with 4-amino-1,8-naphthalimide and light activated. The artery does not exhibit intimal thickening.

### Example 2

The Medtronic Sprinter Legend balloon catheter was used for these studies, which measures 6 x 1.25 mm. The light activation of 10-8-10 PAS is with a blue light laser at 450 nm and 100mW for one minute. The fiber is 0.01" and is fed down the lumen of the catheter. Light fiber #K-004 was used for all rats. The treatment zone is 10 mm. PAS lot 11RO3-11PO1, Ref 70-50007-00 was used in rats 38-57 and Batch 11RO1, 19 Sept 2012, for rats 58-68.

The animal protocol (#94-10-11-14D) has been approved by the USD/VAMC IACUC committee and adheres to all published standards and regulations regarding animal experimentation. The VAMC is an AAALAC accredited animal care facility. Male Sprague-Dawley rats weighing between 344-458 grams (average of 404 grams) were anesthetized with isofluroane gas throughout the experiment. The femoral arteries were exposed with a small incision (1-2 cm). Side vessels were clamped or temporarily tied off. The femoral artery was accessed through a popliteal branch, which was subsequently tied off after surgery. Blood flow as maintained through the other popliteal branch and saphenous artery. Blood was rinsed out of the clamped blood vessel with 1xPBS. A balloon catheter (Medtronic OTW Sprinter Legend, 1.25 x 6 mm, 2.4French, Medtronic, Minneapolis, MN) was inflated and the internal intimal layer was damaged by pulling back on the inflated balloon. This was repeated three times. This is based on a rat model of arterial endothelial cell damage (Gabeler et al, 2002, BMC Cardiovascular Disorders 2:16; Jackson, et al, 1993 Arterioscler Thromb Vasc Biol 13:8; Matsushita et al, 2011, Molecular Biology of the Cell, 22). We use this model of balloon-mediated intimal damage for intimal hyperplasia studies (2 weeks after damage). Immediately after balloon damage, these arteries were then exposed to 5 minutes of PAS or (PBS control) and some were also exposed to 1-minute activation with the blue light.

Four groups were tested: 1) balloon injury plus light activation; 2) balloon injury plus 10-8-10 PAS for 5 minutes; 3) balloon injury plus 10-8-10 PAS for 5 minutes followed by light activation for 1 minute, 4) balloon injury only. The PAS treatment was administered immediately after damage in all cases.

At 2 weeks after surgery, the rat was humanely killed and damaged femoral arteries and the contralateral control, untouched, arteries were collected from each animal. The arteries were dropped in 20% glycerol for 20 minutes and then frozen in OCT compound and stored in the -70°C freezer until sectioning. Arteries were sectioned on a cryostat, set at 7 microns, and placed on Teflon-coated slides with 6 wells, therefore, each slide had 6 sequential sections. The slides were then dried overnight on the benchtop and stored in the -70°C freezer.

The sections were scored/measured as follows: Lines were drawn around the luminal edges of the intimal layer and the resulting area (representing the lumen of the artery) was calculated. Lines were also drawn on the internal and external-most elastic lamina to define the medial layer. The intimal area is calculated subtracting the lumen from the internal elastic lamina, and the medial area is measured by subtracting the internal from the exterior-most elastic lamina (see Figure 1). In addition, four lines were drawn in four locations across both the intimal layer and the medial layer to estimate the thickness of the intima and the medial layers (only one line is drawn in Figure 1 on each layer). The average of the 4 lines is used for the thickness measurement for that section. So 11 measurements were drawn on each section to calculate luminal area, intimal area, medial area, intimal thickness, and medial thickness. These measurements were then averaged for each animal.

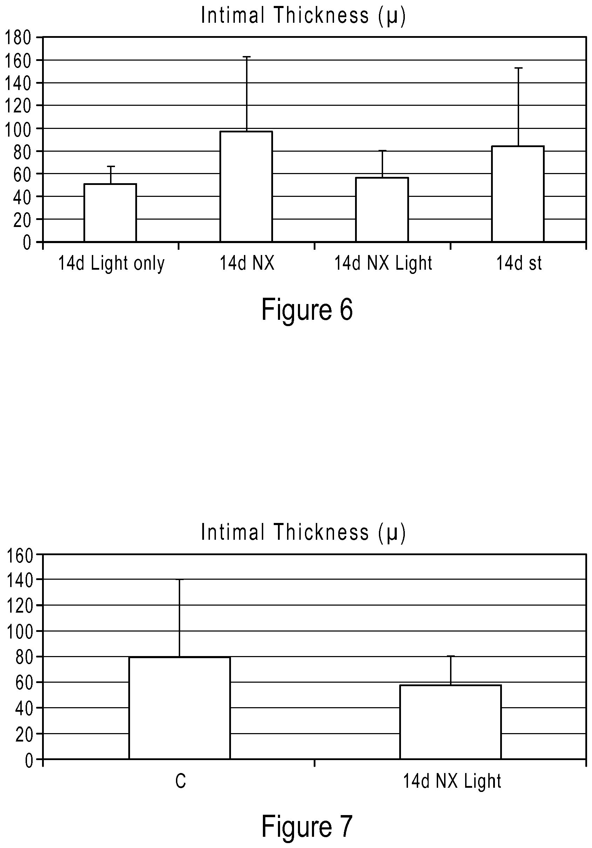

Figure 6 shows the average +/- standard deviation of all four groups. Figure 7 combines the three non-treatment groups into one and compares it to the one treatment group.

Group 3, the inventive group, exhibited a deceased inflammatory response in comparison to Groups 1, 2, and 4, the non-inventive groups. This is evidenced by the increased intimal thickening present in the blood vessels of Groups 1, 2, and 4.

One of ordinary skill in the art can use various techniques known in the art to clinically assess the softening of plaque. For example, a fluoroscope can be used to view the blood vessel with calcified plaque before and after treatment with the disclosed composition. Similarly, computed tomography angiograph (CTA) can be used pre- and post-treatment to observe the changes in the treated blood vessel comprising plaque.

## Claims

1. A composition comprising a 4-amino-1,8-naphthalimide compound for use in a method of decreasing an inflammatory response in a damaged blood vessel, wherein a bolus of a composition comprising a 4-amino-1,8-naphthalimide compound is applied to the damaged blood vessel, wherein the damaged blood vessel is inflamed.

2. The composition for use according to claim 1, further comprising after the step of applying, a step of activating the composition with a sufficient amount of an activating agent, preferably wherein the activating agent is selected from radiated energy, electromagnetic energy, laser, electric current, electrons, thermal neutrons, and chemicals.

3. The composition for use according to claim 1, wherein the damaged blood vessel is an artery or a vein.

4. The composition for use according to claim 1, wherein the damaged blood vessel is damaged as a result of atherosclerosis, a surgical procedure, diabetes, hypertension, autoimmune disease, aneurysm, accident or injury, a minimally-invasive interventional procedure, and repeated access by needle, preferably wherein the surgical procedure comprises at least one of an endarterectomy, vascular graft implant, vascular anastomosis, and bypass graft, preferably wherein the minimally-invasive interventional procedure comprises at least one of PTA, PTCA, vascular stenting, and atherectomy.

5. The composition for use according to claim 1, wherein the composition is provided in a bolus in an amount sufficient to provide a high systemic concentration and/or wherein the compound is applied for a period of time from about 1 second to about 1 hour.

6. The composition for use according to claim 1, wherein the compound includes a dimer or a trimer of naphthalimide rings.

7. The composition for use according to claim 1, wherein the compound includes a dimer of naphthalimide rings.

8. The composition for use according to claim 7, wherein the compound has the general formula (V):

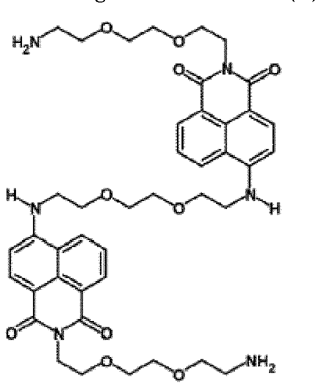

9. The composition for use according to claim 1, wherein an inflammatory response comprises at least one of an increase in intimal thickening, clot formation, increase in concentration of cells, and an increase in mediators, preferably wherein the increase in concentration of cells comprises cells chosen from neutrophils, macrophages, mast cells, helper T cells, NKT cells, B cells, fibroblasts, and VSMCs, preferably wherein the increase of mediators comprises mediators chosen from matrix metalloproteinase-12, matrix metalloproteinase-3, matrix metalloproteinase-9, chymase, tryptase, IFN-γ, IL-4, MIP-1α, IL-β, TGF-β, immunoglobulins, cathepsins, neutral elastase, matrix metalloproteinase-8, VEGF, MCP-1, and matrix metalloproteinase-2.

10. The composition for use according to claim 1, wherein the inflammatory response is an acute inflammatory response.

11. The composition for use according to claim 1, wherein the inflammatory response is a chronic inflammatory response.

12. The composition for use according to claim 1, wherein the inflammatory response is reduced as compared to damaged blood vessel that has not been subjected to application of the 4-amino-1,8-naphthalimide compound.

13. The composition for use according to claim 1, wherein the 4-amino-1,8-naphthalimide compound is dissolved in a solvent.

14. The composition for use according to claim 13, wherein the compound is present in the composition at a concentration of 2 mg/mL.

15. The composition for use according to claim 13, wherein the solvent is phosphate buffered saline, dimethylformamide or isopropyl alcohol.

16. The composition for use according to claim 1, wherein the composition comprises one or more excipients, buffers, carriers, stabilizers, preservatives and/or bulking agents.

17. The composition for use according to claim 1, wherein the composition is in the form of a liquid, a solid, a dispersion, a suspension, a hydrogel, a particle, a nanoparticle, a thin film, or a shaped structure.

18. A composition for use in a method according to claim 1 for decreasing vasodilation in a damaged blood vessel.

19. A composition for use in a method according to claim 1 for decreasing blood supply to a damaged blood vessel.

20. A composition for use in a method according to claim 1 for decreasing permeability of local capillaries of a damaged blood vessel.

21. A composition for use in a method according to claim 1 for decreasing the incidence of restenosis to a damaged blood vessel.

22. The composition for use according to any one of claims 1-3 and 13-17, wherein the compound has the general formula (II) 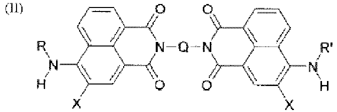 wherein R, R¹ and Q are each selected from the group consisting of straight-chain and branched chain alkyl groups having from 2 to 200 carbons, optionally substituted with one or more ether, amide or amine groups, and wherein X is hydrogen.

## Patentansprüche

1. Zusammensetzung, umfassend eine 4-Amino-1,8-naphthalimid-Verbindung zur Verwendung in einem Verfahren zum Verringern einer Entzündungsreaktion in einem beschädigten Blutgefäß, wobei ein Bolus einer Zusammensetzung, umfassend eine 4-Amino-1,8-naphthalimid-Verbindung, auf das beschädigte Blutgefäß appliziert wird, wobei das beschädigte Blutgefäß entzündet ist.

2. Zusammensetzung zur Verwendung nach Anspruch 1, ferner umfassend nach dem Schritt des Applizierens einen Schritt des Aktivierens der Zusammensetzung mit einer ausreichenden Menge eines Aktivierungsmittels, vorzugsweise wobei das Aktivierungsmittel ausgewählt ist aus abgestrahlter Energie, elektromagnetischer Energie, Laser, elektrischem Strom, Elektronen, thermischen Neutronen, und Chemikalien.

3. Zusammensetzung zur Verwendung nach Anspruch 1, wobei das beschädigte Blutgefäß eine Arterie oder eine Vene ist.

4. Zusammensetzung zur Verwendung nach Anspruch 1, wobei das beschädigte Blutgefäß infolge von Atherosklerose, einem chirurgischen Eingriff, Diabetes, Bluthochdruck, Autoimmunerkrankung, Aneurysma, Unfall oder Verletzung, einem minimal-invasiven Interventionsverfahren, und wiederholtem Zugang durch Nadel beschädigt ist, vorzugsweise wobei das chirurgische Verfahren wenigstens eines von einer Endarteriektomie, einem Gefäßtransplantatimplantat, einer Gefäßanastomose, und einem Bypass-Transplantat umfasst, vorzugsweise wobei das minimal-invasive interventionelle Verfahren wenigstens eines von PTA, PTCA, Gefäßstenting und Atherektomie umfasst.

5. Zusammensetzung zur Verwendung nach Anspruch 1, wobei die Zusammensetzung in einem Bolus in einer Menge bereitgestellt wird, ausreichend, um eine hohe systemische Konzentration bereitzustellen, und/oder wobei die Verbindung für einen Zeitraum von ungefähr 1 Sekunde bis ungefähr 1 Stunde appliziert wird.

6. Zusammensetzung zur Verwendung nach Anspruch 1, wobei die Verbindung ein Dimer oder ein Trimer von Naphthalimidringen einschließt.

7. Zusammensetzung zur Verwendung nach Anspruch 1, wobei die Verbindung ein Dimer von Naphthalimidringen einschließt.

8. Zusammensetzung zur Verwendung nach Anspruch 7, wobei die Verbindung die allgemeine Formel (V) hat:

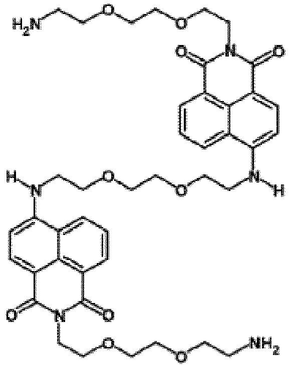

9. Zusammensetzung zur Verwendung nach Anspruch 1, wobei eine Entzündungsreaktion wenigstens eines von einer Zunahme in Intimaverdickung, Gerinnselbildung, Zunahme an Konzentration von Zellen, und Zunahme an Mediatoren umfasst, vorzugsweise wobei die Zunahme an Konzentration von Zellen Zellen gewählt aus Neutrophilen, Makrophagen, Mastzellen, Helfer-T-Zellen, NKT-Zellen, B-Zellen, Fibroblasten, und VSMCs umfasst, vorzugsweise wobei die Zunahme von Mediatoren Mediatoren gewählt aus Matrix-Metalloproteinase-12, Matrix-Metalloproteinase-3, Matrix-Metalloproteinase-9, Chymase, Tryptase, IFN-γ, IL-4, MIP-1α, IL-β, TGF-β, Immunglobulinen, Cathepsinen, neutraler Elastase, Matrix-Metalloproteinase-8, VEGF, MCP-1 und Matrix-Metalloproteinase-2 umfasst.

10. Zusammensetzung zur Verwendung nach Anspruch 1, wobei die Entzündungsreaktion eine akute Entzündungsreaktion ist.

11. Zusammensetzung zur Verwendung nach Anspruch 1, wobei die Entzündungsreaktion eine chronische Entzündungsreaktion ist.

12. Zusammensetzung zur Verwendung nach Anspruch 1, wobei die Entzündungsreaktion im Vergleich zu einem beschädigten Blutgefäß, das nicht Anwendung der 4-Amino-1,8-naphthalimid-Verbindung unterzogen wurde, verringert ist.

13. Zusammensetzung zur Verwendung nach Anspruch 1, wobei die 4-Amino-1,8-naphthalimid-Verbindung in einem Lösungsmittel gelöst ist.

14. Zusammensetzung zur Verwendung nach Anspruch 13, wobei die Verbindung in der Zusammensetzung in einer Konzentration von 2 mg/mL vorhanden ist.

15. Zusammensetzung zur Verwendung nach Anspruch 13, wobei das Lösungsmittel phosphatgepufferte Salzlösung, Dimethylformamid oder Isopropylalkohol ist.

16. Zusammensetzung zur Verwendung nach Anspruch 1, wobei die Zusammensetzung einen oder mehrere Hilfsstoffe, Puffer, Träger, Stabilisatoren, Konservierungsmittel und/oder Füllstoffe umfasst.

17. Zusammensetzung zur Verwendung nach Anspruch 1, wobei die Zusammensetzung in der Form einer Flüssigkeit, eines Feststoffs, einer Dispersion, einer Suspension, eines Hydrogels, eines Partikels, eines Nanopartikels, eines dünnen Films, oder einer geformten Struktur ist.

18. Zusammensetzung zur Verwendung in einem Verfahren nach Anspruch 1 zum Verringern von Vasodilatation in einem beschädigten Blutgefäß.

19. Zusammensetzung zur Verwendung in einem Verfahren nach Anspruch 1 zum Verringern von Blutversorgung an ein beschädigtes Blutgefäß.

20. Zusammensetzung zur Verwendung in einem Verfahren nach Anspruch 1 zum Verringern von Permeabilität lokaler Kapillaren eines beschädigten Blutgefäßes.

21. Zusammensetzung zur Verwendung in einem Verfahren nach Anspruch 1 zum Verringern des Auftretens von Restenose an einem beschädigten Blutgefäß.

22. Zusammensetzung zur Verwendung nach einem der Ansprüche 1 - 3 und 13 - 17, wobei die Verbindung die allgemeine Formel (II) hat 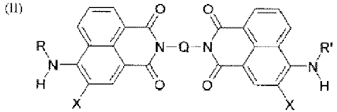 wobei R, R¹ und Q jeweils ausgewählt sind aus der Gruppe bestehend aus geradkettigen und verzweigtkettigen Alkylgruppen mit von 2 bis 200 Kohlenstoffen, optional substituiert mit einer oder mehreren Ether-, Amid- oder Amingruppen, und wobei X Wasserstoff ist.

## Revendications

1. Composition comprenant un composé de 4-amino-1,8-naphtalimide à utiliser dans un procédé de diminution d'une réponse inflammatoire dans un vaisseau sanguin endommagé, dans laquelle un bolus d'une composition comprenant un composé de 4-amino-1,8-naphtalimide est appliqué au vaisseau sanguin endommagé, dans lequel le vaisseau sanguin endommagé est enflammé.

2. Composition à utiliser selon la revendication 1, comprenant en outre, après l'étape d'application, une étape d'activation de la composition avec une quantité suffisante d'un agent d'activation, de préférence dans laquelle l'agent d'activation est choisi parmi de l'énergie rayonnée, de l'énergie électromagnétique, du laser, du courant électrique, des électrons, des neutrons thermiques et des produits chimiques.

3. Composition à utiliser selon la revendication 1, dans laquelle le vaisseau sanguin endommagé est une artère ou une veine.

4. Composition à utiliser selon la revendication 1, dans laquelle le vaisseau sanguin endommagé est endommagé à la suite d'une athérosclérose, d'une intervention chirurgicale, du diabète, de l'hypertension, d'une maladie autoimmune, d'un anévrisme, d'un accident ou d'une blessure, d'une procédure interventionnelle nvasive au minimum et d'un accès répété par une aiguille, de préférence dans laquelle la procédure chirurgicale comprend au moins l'un d'une endartériectomie, d'un implant de greffe vasculaire, d'une anastomose vasculaire et d'une greffe de pontage, de préférence dans laquelle la procédure interventionnelle invasive au minimum comprend au moins une parmi une PTA, une PTCA, une pose d'endoprothèse vasculaire et une athérectomie.

5. Composition à utiliser selon la revendication 1, dans laquelle la composition est fournie dans un bolus en une quantité suffisante pour fournir une concentration systémique élevée et/ou dans laquelle le composé est appliqué pendant une période de temps d'environ 1 seconde à environ 1 heure.

6. Composition à utiliser selon la revendication 1, dans laquelle le composé comprend un dimère ou un trimère de cycles de naphtalimide.

7. Composition à utiliser selon la revendication 1, dans laquelle le composé comprend un dimère de cycles de naphtalimide.

8. Composition à utiliser selon la revendication 7, dans laquelle le composé a la formule générale (V) :

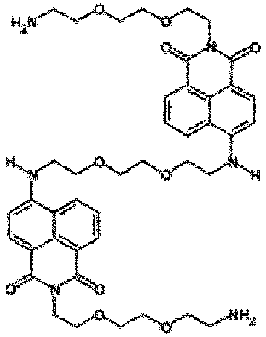

9. Composition à utiliser selon la revendication 1, dans laquelle une réponse inflammatoire comprend au moins l'une d'une augmentation de l'épaississement de l'intima, d'une formation de caillot, d'une augmentation de la concentration de cellules, et d'une augmentation de médiateurs, de préférence dans laquelle l'augmentation de la concentration de cellules comprend des cellules choisies parmi des neutrophiles, des macrophages, des mastocytes, des cellules T auxiliaires, des cellules NKT, des cellules B, des fibroblastes et des VSMC, de préférence dans laquelle l'augmentation de médiateurs comprend des médiateurs choisis parmi la métalloprotéinase matricielle-12, la métalloprotéinase matricielle-3, la métalloprotéinase matricielle-9, la chymase, tryptase, IFN-γ, IL-4, MIP-1α, IL-β, TGF-β, des immunoglobulines, des cathepsines, de l'élastase neutre, la métalloprotéinase matricielle-8, VEGF, MCP-1 et la métalloprotéinase matricielle-2.

10. Composition à utiliser selon la revendication 1, dans laquelle la réponse inflammatoire est une réponse inflammatoire aiguë.

11. Composition à utiliser selon la revendication 1, dans laquelle la réponse inflammatoire est une réponse inflammatoire chronique.

12. Composition à utiliser selon la revendication 1, dans laquelle la réponse inflammatoire est réduite par rapport à un vaisseau sanguin endommagé qui n'a pas été soumis à une application du composé de 4-amino-1,8-naphtalimide.

13. Composition à utiliser selon la revendication 1, dans laquelle le composé de 4-amino-1,8-naphtalimide est dissous dans un solvant.

14. Composition à utiliser selon la revendication 13, dans laquelle le composé est présent dans la composition à une concentration de 2 mg/mL.

15. Composition à utiliser selon la revendication 13, dans laquelle le solvant est une solution saline tamponnée au phosphate, du diméthylformamide ou de l'alcool isopropylique.

16. Composition à utiliser selon la revendication 1, dans laquelle la composition comprend un ou plusieurs excipients, tampons, supports, stabilisants, conservateurs et/ou agents gonflants.

17. Composition à utiliser selon la revendication 1, dans laquelle la composition est sous la forme d'un liquide, d'un solide, d'une dispersion, d'une suspension, d'un hydrogel, d'une particule, d'une nanoparticule, d'un film mince ou d'une structure mise en forme.

18. Composition à utiliser dans un procédé selon la revendication 1 pour diminuer une vasodilatation dans un vaisseau sanguin endommagé.

19. Composition à utiliser dans un procédé selon la revendication 1 pour diminuer un apport sanguin vers un vaisseau sanguin endommagé.

20. Composition à utiliser dans un procédé selon la revendication 1 pour diminuer la perméabilité de capillaires locaux d'un vaisseau sanguin endommagé.

21. Composition à utiliser dans un procédé selon la revendication 1 pour diminuer l'incidence d'une resténose à un vaisseau sanguin endommagé.

22. Composition à utiliser selon l'une quelconque des revendication 1 à 3 et 13 à 17, dans laquelle le composé a la formule générale (II) 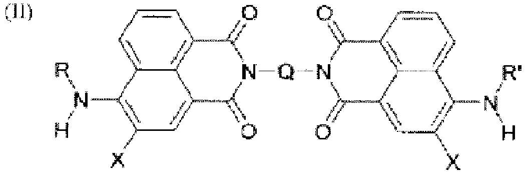 dans laquelle R, R¹ et Q sont chacun choisis dans le groupe constitué de groupes alkyle à chaîne droite et à chaîne ramifiée ayant de 2 à 200 carbones, éventuellement substitués par un ou plusieurs groupes éther, amide ou aminé, et dans laquelle X est l'hydrogène.
